(19) Europäisches Patentamt
European Patent Office
Office européen des brevets

(11) Veröffentlichungsnummer: **0 457 102 A2**

(12) **EUROPÄISCHE PATENTANMELDUNG**

(21) Anmeldenummer: **91107056.3**

(22) Anmeldetag: **01.05.91**

(51) Int. Cl.[5]: **C07C 69/732**, C07C 67/343, C07C 67/04, C08G 64/06, C08G 63/193

(30) Priorität: **15.05.90 DE 4015541**

(43) Veröffentlichungstag der Anmeldung: **21.11.91 Patentblatt 91/47**

(84) Benannte Vertragsstaaten: **BE DE FR GB IT NL**

(71) Anmelder: **BAYER AG**
**W-5090 Leverkusen 1 Bayerwerk(DE)**

(72) Erfinder: **Westeppe, Uwe, Dr.**
**Vogelkamp 72**
**W-4020 Mettmann(DE)**
Erfinder: **Fengler, Gerd, Dr.**
**Deutschordensweg 12**
**W-4150 Krefeld(DE)**
Erfinder: **Serini, Volker, Dr.**
**Sebastian-Kneipp-Weg 2**
**W-4150 Krefeld(DE)**

# (54) tert.-Alkylester-Bisphenole, Verfahren zu ihrer Herstellung und ihre Verwendung.

(57) Gegenstand der Erfindung sind neue, seitenständig tert.-Alkylestergruppen aufweisende Bisphenole (I) und ihre Verwendung zur Herstellung von aromatischen Polyestern, Polycarbonaten und (Co)Polyestercarbonaten,

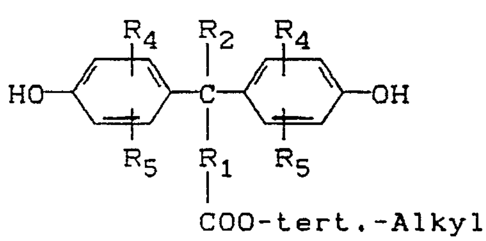

(I)

vorzugsweise ist

| | |
|---|---|
| $R_1$ | $-CH_2-CH_2-$, |
| $R_2$ | $CH_3$, |
| $R_4$ und $R_5$ | H oder Methyl. |

EP 0 457 102 A2

Gegenstand der Erfindung sind neue, seitenständig tert.-Alkylestergruppen aufweisende Bisphenole (I), ein Verfahren zu ihrer Herstellung, ferner ihre Verwendung zur Herstellung aromatischer Polycarbonate, aromatischer Polyester und aromatischer (Co)Polyestercarbonate auf Basis dieser Bisphenole. Weiterer Erfindungsgegenstand sind die entsprechenden aromatischen Polycarbonate, aromatischen Polyester und die Copolyestercarbonate auf Basis dieser Bisphenole (I).

Die neuen Bisphenole sind durch folgende Formel gekennzeichnet,

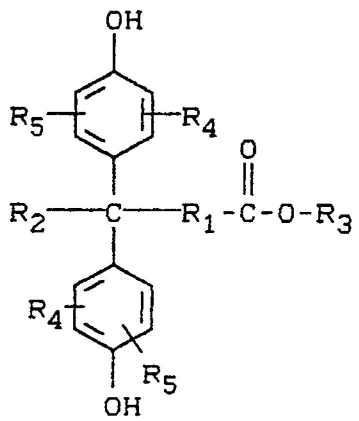

(I),

wobei

- $R_1$ ein geradkettiger oder verzweigter $C_1$-$C_6$-Alkylenrest, bevorzugt $(CH_2)_n$;
- $R_2$ ein $C_1$-$C_{12}$-Alkylrest, bevorzugt $CH_3$,
- $R_3$ ein tertiärer $C_4$-$C_9$-Alkylrest, bevorzugt ein tert.-butyl-, tert.-amyl- oder -octylrest,
- $R_4$, $R_5$ unabhängig voneinander Wasserstoff, Halogen, $C_1$-$C_6$-Alkyl, Cycloalkyl, Aryl und
- n eine ganze Zahl von 0 bis 6, bevorzugt 1 bis 3, insbesondere 2

bedeuten.

Primäre und sekundäre Alkylester der Formel (I) ($R_3$ = primärer oder sekundärer Alkylrest) sind bekannt (vgl. US 2 933 520, US 2 984 685, US 3 296 160, US 3 427 345).

Ferner ist aus US 3 291 774 bekannt, Polycarbonate auf Basis von Diphenolcarbonsäuren in wäßriger Phase herzustellen, Polycarbonate auf Basis primärer und sekundärer Alkylester der Formel (I) ($R_3$ = primärer oder sekundärer Alkylrest) sind ebenfalls bekannt (J. App. Polymer Sci. 10, 245 (1966)). Sie werden nach dem sogenannten Pyridinverfahren hergestellt, die Polycarbonate können nicht nach dem Verfahren der Phasengrenzflächenpolykondensation hergestellt werden, da die Ester unter den Reaktionsbedingungen verseifen und dann als trifunktionelle Verbindungen zu verzweigten bzw. vernetzten Produkten führen.

Bisphenole, die primäre und sekundäre Alkylestergruppen aufweisen, sind auch aus J. Polymer Sci. part A-1, Vol. 8, S. 1419 (1970) bekannt. Auch hier wurden die Polycarbonate nach dem Pyridinverfahren hergestellt. Für technische Anwendungen ist das Pyridinverfahren aus ökologischen und ökonomischen Gründen zu aufwendig.

Aus EP-A-312 811 sind carboxylfunktionalisierte Polycarbonate anderer Struktur bekannt. Sie werden durch Einsatz spezieller Kettenabbrecher vonm Typ Hydroxyphenylcarbonsäuren oder ihrer tert.-Butylester, -Imide oder -Anhydride synthetisiert. Die nach EP-A-312 811 erhältlichen Polycarbonate enthalten somit maximal 2 tert.-Butylestergruppen direkt am Phenylrest und jeweils am Ende der Molekülkette.

Zur Herstellung von Polymeren deutlich modifizierter Struktur ist es günstiger, die Anzahl von pro Molekülkette vorhandenen tert.-Alkylestergruppen darüber hinaus variieren zu können und somit auch mehr als zwei Alkylestergruppen pro Molekülkette, insbesondere aber auch innerhalb der Molekülkette, zur Verfügung zu haben. Derartige erfindungsgemäße Polymeren eignen sich dann insbesondere zur Verwendung in Polymerblends, entsprechend der eigenen, nicht vorveröffentlichten Deutschen Patentanmeldung (am gleichen Tag eingereichten Deutschen Patentanmeldung Le A 27 122 (P4015540.4)).

Technische Aufgabe war es daher, mehrfach seitenständige tert.-Alkylestergruppierungen tragende Poly(ester)carbonate bzw. Polyester, bevorzugt nach dem Phasengrenzflächenverfahren, herzustellen, wobei die neuen, erfindungsgemäßen Polymerisate nicht bei der Herstellung vernetzen und thermoplastisch weiterverarbeitbar bleiben.

Es wurde überraschenderweise gefunden, daß die erfindungsgemäßen Dihydroxyverbindungen der Formel (I) zur Herstellung von hochmolekularen thermoplastischen Polyestern, Polycarbonaten oder (Co)-

Polyestercarbonaten auch nach dem Phasengrenzflächenverfahren eingesetzt werden können, wenn der Rest $R_3$ ein tert.-$C_4$-$C_9$-Alkylrest ist.

Gegenstand der Erfindung sind somit die neuen seitenständigen tert.-Alkylestergruppen aufweisenden Bisphenole der Formel (I)

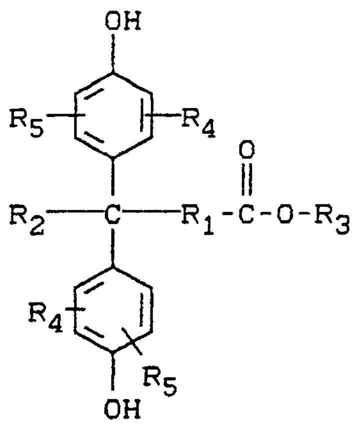

(I),

Insbesondere sind aus den beanspruchten Bisphenolen der Formel (I) folgende Bisphenole der Formeln (II) bis (IV) bevorzugt

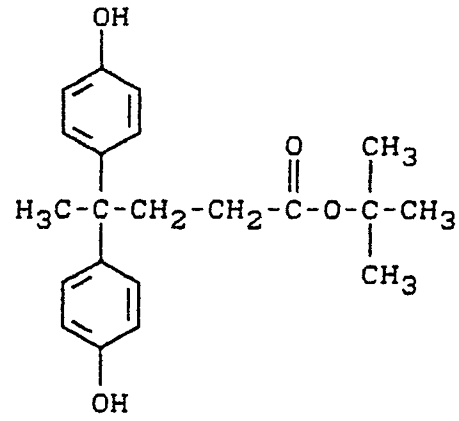

(II),

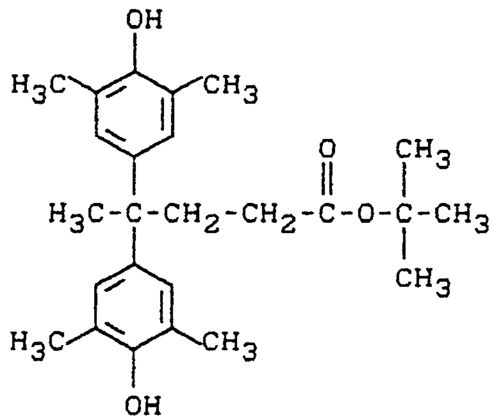

(III) und

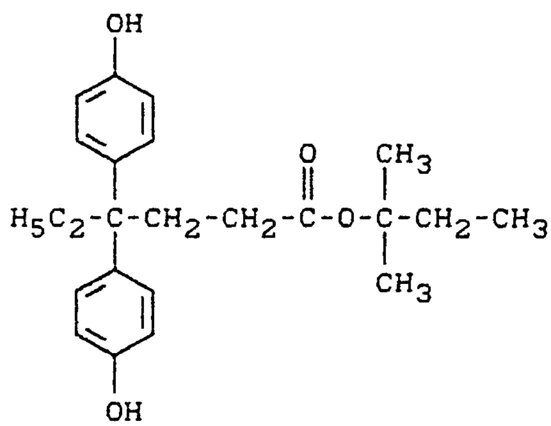

(IV),

wobei der 4,4-Bis-(4-hydroxyphenyl)-valeriansäure-tert.-butylester (Formel (II)) besonders bevorzugt ist.

Die erfindungsgemäßen tert.-Alkylesterbisphenole der Formel (I) können in an sich bekannter Weise durch Veresterung von Bisphenolcarbonsäuren ((I), $R_3$ = H) mit $\alpha,\alpha$-disubstituierten Olefinen hergestellt werden (zur Herstellung von Estern tert. Alkohole vgl. Houben-Weyl: "Methoden der Organischen Chemie", Band Sauerstoffverbindungen III, G. Thieme Verlag Stuttgart 1952, S. 534, 550).

Beispiele für geeignete Bisphenolcarbonsäuren sind: 4,4-Bis-(4-hydroxyphenyl)-valeriansäure, 4,4-Bis-(4-hydroxyphenyl)-capronsäure, 5,5-Bis-(4-hydroxy-3,5-dimethyl)-heptansäure, 5,5-Bis-(4-hydroxyphenyl)-heptansäure, 4,4-Bis-(4-hydroxyphenyl)-octansäure.

Aus Olefine zur Herstellung der Verbindungen (I) können eingesetzt werden:

Isobuten, 2-Methyl-1-buten, 2,3-Dimethyl-1-buten, 2-Ethyl-1-buten, 2,3,3-Trimethylbuten-1, 2,3-Dimethylpenten-1, 2,4-Dimethylpenten-1, 2-Ethyl-1-penten, 2-Methyl-1-penten. Die Herstellung der Verbindungen (I) erfolgt üblicherweise unter Zusatz von sauren Katalysatoren wie Chlorwasserstoff, Bromwasserstoff, Bortrifluorid, Phosphorsäure, konzentrierte Salzsäure oder Schwefelsäure sowie Mischungen aus Essigsäure und Acetanhydrid.

Es können auch saure Ionenaustauscher verwendet werden.

Die neuen, erfindungsgemäßen, seitenständigen tert.-Alkylestergruppen enthaltenden Bisphenole der Formel (I) können ferner auch in an sich bekannter Reaktionsweise durch Umsetzung von Phenolen der Formel (V)

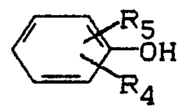

(V)

mit Ketocarbonsäureestern der Formel (VI)

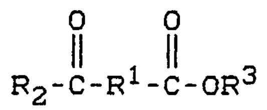

(VI)

hergestellt werden, wobei in den Formeln (V) und (VI) $R_1$, $R_2$, $R_3$, $R_4$, $R_5$ und n die für Formel (I) angegebene Bedeutung haben.

Die Phenole der Formel (V) sind entweder literaturbekannt oder nach literaturbekannten Verfahren erhältlich (siehe beispielsweise für Kresole und Xylenole, Ullmanns Encyklopädie der technischen Chemie 4. neubearbeitete und erweitere Auflage Band 15, Seiten 61 - 77, Verlag Chemie-Weinheim-New York 1978; für Chlorphenole, Ullmanns Encyklopädie der technischen Chemie, 4. Auflage, Verlag Chemie, 1975, Band 9, Seiten 573-582; und für Alkylphenole, Ullmanns Encyklopädie der technischen Chemie, 4. Auflage, Verlag Chemie 1979, Band 18, Seiten 191-214).

Beispiele für geeignete Phenole der Formel (V) sind: Phenol, o-Kresol, m-Kresol, 2,6-Dimethylphenol, 2-Chlorphenol, 3-Chlorphenol, 2,6-Dichlorphenol, 2-Cyclohexylphenol, 2,6-Diphenylphenol und o-Benzylphenol.

Beispiele für Ketocarbonsäureester der Formel (VI) sind:
3-Oxo-buttersäure-tert.-butylester, 2-Methyl-3-oxo-valeriansäure-tert.-butylester, Lävulinsäure-tert.-butylester, 5-Oxoheptansäure-tert.-butylester, 5-Oxoheptansäure-tert.-butylester, 4-Oxohexansäure-tert.-butylester, 5-Oxohexansäure-tert.-butylester, 5-Oxoheptansäure-tert.-pentylester, 4-Oxoheptansäure-tert.-octylester, 4-Oxohexansäure-tert.-hexylester, 5-Oxohexansäure-tert.-hexylester.

Zur Bisphenolherstellung werden im allgemeinen 2 bis 10 Mol, vorzugsweise 2,5 bis 6 Mol Phenol (V) pro Mol Ketocarbonsäureester (VI) verwendet, Bevorzugte Reaktionszeiten betragen 1 bis 100 Stunden. Im allgemeinen arbeitet man bei Temperaturen von -30 °C bis 300 °C, vorzugsweise von -15 °C bis 150 °C und bei Drücken von 1 bis 20 bar, vorzugsweise 1 bis 10 bar.

Die Kondensation wird im allgemeinen in Gegenwart saurer Katalysatoren durchgeführt. Beispiele sind Chlorwasserstoff, Bromwasserstoff, Fluorwasserstoff, Bortrifluorid, Aluminiumtrichlorid, Phosphorpentoxid, Phosphorsäure, konzentrierte Salzsäure oder Schwefelsäure sowie Mischungen aus Essigsäure und Acetanhydrid. Die Verwendung saurer Ionenaustauscher ist ebenfalls möglich.

Weiterhin kann die Umsetzung durch Zugabe von Co-Katalysatoren wie $C_1$-$C_{18}$-Alkylmercaptanen, Schwefelwasserstoff, Thiophenolen, Thiosäuren und Dialkylsulfiden beschleunigt werden.

Die Kondensation kann ohne Lösungsmittel oder in Gegenwart eines inerten Lösungsmittels (z.B. aliphatischer und aromatischer Kohlenwasserstoff, Chlorkohlenwasserstoff) durchgeführt werden.

In den Fällen, in denen der Katalysator gleichzeitig als wasserentziehendes Mittel fungiert, ist es nicht erforderlich, zusätzlich wasserentziehende Mittel einzusetzen, letzteres ist jedoch zur Erzielung guter Umsätze in jedem Fall dann vorteilhaft, wenn der eingesetzte Katalysator das Reaktionswasser nicht bindet.

Geeignete wasserentziehende Mittel sind beispielsweise Acetanhydrid, Zeolithe, Polyphosphorsäure und Phosphorpentoxid.

Gegenstand der Erfindung ist somit auch ein Verfahren zur Herstellung der neuen Bisphenole mit seitenständigen tert.-Alkylestergruppen, entsprechend der Formel (I), dadurch gekennzeichnet, daß Bisphenolcarbonsäuren ((I), $R^3$ = H) mit Olefinen unter Zusatz von sauren Katalysatoren bei Temperaturen von -30 °C bis 200 °C, vorzugsweise von -15 °C bis 120 °C unter Drücken von 1 bis 100 bar, vorzugsweise von 1 bis 50 bar, in Gegenwart saurer Katalysatoren und gegebenenfalls in Gegenwart von Lösungsmitteln und/oder dehydratisierenden Agentien umgesetzt werden oder daß Ketocarbonsäureester (VI) mit Phenolen

(V) unter Zusatz von sauren Katalysatoren umgesetzt werden. Das Verfahren der Herstellung über Bisphenolcarbonsäuren ist dabei bevorzugt.

Die erfindungsgemäßen Bisphenole der Formel (I) sind insbesondere geeignet zur Herstellung von hochmolekularen aromatischen Polyestern, aromatischen Polycarbonaten oder (Co)Polyestercarbonaten, bevorzugt in einem Syntheseverfahren nach dem Phasengrenzflächenverfahren, welches zu weitgehend unverzweigten Polymeren führt.

Die neuen Polyester, Polycarbonate und (Co)Polyestercarbonate sind gut verarbeitbare Thermoplasten, die eine Reihe von günstigen Eigenschaften gegenüber entsprechenden Polymeren ohne Einbau der neuen Bisphenole (I) zeigen. Weitere neue und unerwartet technische Verwendungen werden in der gleichzeitig eingereichten deutschen Patentanmeldung Le A 27 122 (P 4015514.4) beschrieben.

Gegenstand der Erfindung ist somit auch die Verwendung der Bisphenole der Formel (I) sowie gegebenenfalls anderer Diphenole der Formel

HO-Z-OH (VII)

in einem Verfahren zur Herstellung von hochmolekularen, aromatischen Polyestern,
auf Basis der erfindungsgemäßen Bisphenole (I) sind (zumindest überwiegend)

- aromatische Polycarbonsäuren (bevorzugt Dicarbonsäuren) bzw. reaktiven Derivate (Säurechloride), und/oder
  Polycarbonaten, auf Basis der Bisphenolen (I), gegebenenfalls weiteren Diphenolen (VII) und Phosgen oder einem anderen Carbonatgruppenspender wie Diarylcarbonaten oder Bischlorkohlensäureestern, und/oder
  (CO)Polyestercarbonaten, auf der Basis von Bisphenolen (I), gegebenenfalls weiteren Diphenolen (VII), aromatischen Dicarbonsäuren bzw. reaktiven Derivate (Säurechloride) und Phosgen, Diarylcarbonat oder Bischlorkohlensäureestern

jeweils - soweit erwünscht - in Gegenwart kleiner Mengen an Kettenabbrechern
Es können dabei sowohl ein erfindungsgemäßes Bisphenol der Formel (I) unter Bildung von Homopolycarbonaten als auch mehrere Bisphenole der Formel (I) und gegebenenfalls weitere Diphenole unter Bildung von Copolycarbonaten verwendet werden.

Bevorzugt werden die Bisphenole der Formel (I) jedoch im Gemisch mit an sich üblichen, anderen Diphenolen, beispielsweise mit denen der Formel

HO-Z-OH (VII),

zur Herstellung von hochmolekularen, aromatischen Copolycarbonaten, aromatischen Polyestern, aromatischen Polyestercarbonaten verwendet.

Geeignete andere Diphenole der Formel HO-Z-OH (VII) sind insbesondere solche, in denen Z ein aromatischer Rest mit 6 bis 30 C-Atomen ist, der einen oder mehrere aromatische Kerne enthalten kann, substituiert sein kann und aliphatische Reste oder andere cycloaliphatische Reste als die der Formel (I) oder Heteroatome als Brückenglieder enthalten kann.

Beispiele für andere Diphenole der Formel (VII) sind:
Hydrochinon,
Resorcin,
Dihydroxydiphenyle,
Bis-(hydroxyphenyl)-alkane,
Bis-(hydroxyphenyl)-cycloalkane,
Bis-(hydroxyphenyl)-sulfide,
Bis-(hydroxyphenyl)-ether,
Bis-(hydroxyphenyl)-ketone,
Bis-(hydroxyphenyl)-sulfone,
Bis-(hydroxyphenyl)-sulfoxide,
α,α'-Bis-(hydroxyphenyl)-diisopropylbenzole
sowie deren kernalkylierte und kernhalogenierte Verbindungen.

Diese und weitere andere geeignete Diphenole sind z.B. in den US-PS 3 028 365, 2 999 835, 3 148 172, 3 275 601, 2 991 273, 3 271 367, 3 062 781, 2 970 131 und 2 999 846, in den deutschen Offenlegungsschriften 1 570 703, 2 063 050, 2 063 052, 2 211 096, 3 832 396, der französischen Patentschrift 1 561 518 und in der Monographie "H. Schnell, Chemistry and Physics of Polycarbonates, Interscience Publishers, New York 1964", beschrieben.

Bevorzugte andere Diphenole sind beispielsweise:
4,4'-Dihydroxydiphenyl,
2,2-Bis-(4-hydroxyphenyl)-propan,
2,4-Bis-(4-hydroxyphenyl)-2-methylbutan,
1,1-Bis-(4-hydroxyphenyl)-cyclohexan,
$\alpha,\alpha'$-Bis-(4-hydroxyphenyl)-p-diisopropylbenzol,
2,2-Bis-(3-methyl-4-hydroxyphenyl)-propan,
2,2-Bis-(3-chlor-4-hydroxyphenyl)-propan,
Bis-(3,5-dimethyl-4-hydroxyphenyl)-methan,
2,2-Bis-(3,5-dimethyl-4-hydroxyphenyl)-propan,
Bis-(3,5-dimethyl-4-hydroxyphenyl)-sulfon,
2,4-Bis-(3,5-dimethyl-4-hydroxyphenyl)-2-methylbutan,
1,1-Bis-(3,5-dimethyl-4-hydroxyphenyl)-cyclohexan,
1,1-Bis-(4-hydroxyphenyl)-3,3,5-trimethylcyclohexan,
1,1-Bis-(4-hydroxyphenyl)-3,3-dimethylcyclohexan,
1,1-Bis-(4-hydroxyphenyl)-3,5-dimethylcyclohexan,
1,1-Bis-(4-hydroxyphenyl)-3-methylcyclohexan,
$\alpha,\alpha'$-Bis-(3,5-dimethyl-4-hydroxyphenyl)-p-diisopropylbenzol,
2,2-Bis-(3,5-dichlor-4-hydroxyphenyl)-propan und
2,2-Bis-(3,5-dibrom-4-hydroxyphenyl)-propan.

Besonders bevorzugte Diphenole der Formel (VII) sind beispielsweise:
2,2-Bis-(4-hydroxyphenyl)-propan,
2,2-Bis-(3,5-dimethyl-4-hydroxyphenyl)-propan,
2,2-Bis-(3,5-dichlor-4-hydroxyphenyl)-propan,
2,2-Bis-(3,5-dibrom-4-hydroxyphenyl)-propan,
1,1-Bis-(4-hydroxyphenyl)-cyclohexan und
1,1-Bis-(4-hydroxyphenyl)-3,3,5-trimethylcyclohexan.

Insbesondere sind 2,2-Bis-(4-hydroxyphenyl)-propan und 1,1-Bis-(4-hydroxyphenyl)-3,3,5-trimethylcyclohexan bevorzugt.

Die anderen Diphenole können sowohl einzeln als auch im Gemisch eingesetzt werden.

Das molare Verhältnis von erfindungsgemäß zu verwendenden Bisphenolen der Formel (I) zu den gegebenenfalls mitzuverwendenden anderen Diphenolen (c1), beispielsweise denen der Formel (VII), soll 0,5 bis 100 % (I) (erfindungsgemäßes Bisphenol) und 99,5 bis 0 Mol-% normales, anderes Diphenol (c1),
vorzugsweise 1 bis 50 Mol-% (I),
99 bis 50 Mol-% anderes, normales Diphenol (c1),
insbesondere 1,5 bis 30 Mol-% (I) und
98,5 bis 70 Mol-% anderes Diphenol (c1)
und ganz besonders bevorzugt 2 bis 20 Mol-% (I) und
98 bis 80 Mol-% anderes Diphenol (c1)
betragen.

Die hochmolekularen Polycarbonate aus den erfindungsgemäßen Bisphenolen der Formel (I), gegebenenfalls in Kombination mit anderen Diphenolen (c1) können nach den bekannten Polycarbonat-Herstellungsverfahren hergestellt werden. Dabei können die verschiedenen Diphenole (I) bzw. (c1) sowohl statistisch als auch blockweise miteinander verknüpft sein.

Gegenstand der Erfindung ist somit insbesondere die Verwendung von (I) in einem Verfahren zur Herstellung von hochmolekularen thermoplastischen, aromatischen Polycarbonaten aus Diphenolen, gegebenenfalls Kettenabbrechern und gegebenenfalls Verzweigern nach den bekannten Methoden der Polycarbonatherstellung, vorzugsweise nach dem Zweiphasengrenzflächenverfahren, das dadurch gekennzeichnet ist, daß man als Diphenole solche der Formel (I) in Mengen von 100 Mol-% bis 0,5 Mol-%, vorzugsweise in Mengen von 50 Mol-% bis 1 Mol-% und insbesondere in Mengen von 30 Mol-% bis 1,5 Mol-% und ganz besonders 20 Mol-% bis 2 Mol-%, bezogen jeweils auf die Gesamtmolmenge an eingesetzten Diphenolen, verwendet.

Als Kettenabbrecher zur Regelung des Molekulargewichtes dienen in bekannter Weise monofunktionelle Verbindungen in üblichen Konzentrationen. Geeignete Verbindungen sind z.B. Phenol, tert.-Butylphenole oder andere Alkyl-$C_1$-$C_7$-substituierte Phenole. Zur Regelung des Molekulargewichtes sind insbesondere kleine Mengen Phenole der Formel (VIII) geeignet

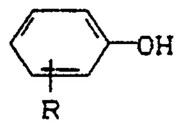
(VIII)

worin R einen verzeigten $C_8$- und/oder $C_9$-Alkylrest darstellt. Bevorzugt ist im Alkylrest R der Anteil an $CH_3$-Protonen zwischen 47 und 89 % und der Anteil der CH- und $CH_2$-Protonen zwischen 53 und 11 %; ebenfalls bevorzugt ist R in o- und/oder p-Stellung zur OH-Gruppe, und besonders bevorzugt die obere Grenze des ortho-Anteils 20 %. Die Kettenabbrecher werden im allgemeinen in Mengen von 0,5 bis 10, bevorzugt 1,5 bis 8 Mol-%, bezogen auf eingesetzte Diphenole, eingesetzt.

Die erfindungsgemäßen Polycarbonate können vorzugsweise nach dem Phasengrenzflächenverfahren (vgl. H. Schnell, "Chemistry and Physics of Polycarbonates", Polymer Reviews, Vol. IX, Seite 33 ff., Interscience Publ., 1964) in an sich bekannter Weise hergestellt werden, Hierbei werden die Bisphenole der Formel (I) in wäßrig alkalischer Phase gelöst. Zur Herstellung von Co-Polycarbonaten mit anderen Diphenolen werden Gemische von Bisphenolen der Formel (I) und den anderen Diphenolen, beispielsweise denen der Formel (VII), eingesetzt. Zur Regulierung des Molekulargewichtes können Kettenabbrecher z.B. der Formel (VIII) zugegeben werden. Dann wird in Gegenwart einer inerten, vorzugsweise Polycarbonat lösenden, organischen Phase mit Phosgen nach der Methode der Phasengrenzflächenkondensation umgesetzt. Die Reaktionstemperatur liegt zwischen 0° C und 40° C.

Als Verzweigter dienen, falls benutzt, in bekannter Weise geringe Mengen, vorzugsweise Mengen zwischen 0,05 und 2,0 Mol-% (bezogen auf eingesetzte Diphenole), an drei oder mehr als dreifunktionellen Verbindungen, insbesondere solchen mit drei oder mehr als drei phenolischen Hydroxylgruppen, um verzweigte Polycarbonate zu erhalten. Einige der verwendbaren Verbindungen mit drei oder mehr als drei phenolischen Hydroxylgruppen sind Phloroglucin,
4,6-Dimethyl-2,4,6-tri-(4-hydroxyphenyl)-hepten-2,
3,6-Dimethyl-2,4,6-tri-(4-hydroxyphenyl)-heptan,
1,3,5-Tri-(4-hydroxyphenyl)-benzol,
1,1,1-Tri-(4-hydroxyphenyl)-ethan,
Tri-(4-hydroxyphenyl)-phenylmethan,
2,2-Bis-(4,4-bis-(4-hydroxyphenyl)-cyclohexyl)-propan,
2,4-Bis-(4-hydroxyphenyl-isopropyl)-phenol,
2,6-Bis-(4-hydroxy-5'-methyl-benzyl)-4-methylphenol,
2-(4-Hydroxyphenyl)-2-(2,4-dihydroxyphenyl)-propan,
Hexa-(4-(4-hydroxyphenyl-isopropyl)-phenyl)-orthoterephthal-säureester,
Tetra-(4-hydroxyphenyl)-methan,
Tetra-(4-(4-hydroxyphenyl-isopropyl)-phenoxy)-methan
und
1,4-Bis-((4',4"-dihydroxytriphenyl)-methyl)-benzol.

Einige der sonstigen dreifunktionellen Verbindungen sind 2,4-Dihydroxybenzoesäure, Trimesinsäure, Cyanurchlorid und 3,3-Bis-(3-methyl-4-hydroxyphenyl)-2-oxo-2,3-dihydro-indol.

Die gegebenenfalls mitzuverwendenden 0,05 bis 2 Mol-% an Verzweigern können entweder mit den Diphenolen in der wäßrig alkalischen Phase vorgelegt werden oder in dem organischen Lösungsmittel gelöst vor der Phosgenierung zugegeben werden.

Neben den einzusetzenden Bisphenolen der Formel (I) sowie den anderen Diphenolen (VII) können auch deren Mono- und/oder Bischlorkohlensäureester mitverwendet werden, wobei diese in organischen Lösungsmitteln gelöst zugegeben werden. Die Menge an Kettenabbrechern sowie an Verzweigern richtet sich dann nach Molen Bisphenolat-Struktureinheiten von (I) und gegebenenfalls von den anderen Diphenolen, wie beispielsweise von (VII); ebenso kann bei Einsatz von Chlorkohlensäureestern die Phosgenmenge in bekannter Weise entsprechend reduziert werden.

Geeignete organische Lösungsmittel für die Lösung der Kettenabbrecher sowie gegebenenfalls für die Verzweiger und die Chlorkohlensäureester sind beispielsweise Methylenchlorid, Chlorbenzol, Aceton, Acetonitril sowie Mischungen dieser Lösungsmittel, insbesondere Mischungen aus Methylenchlorid und Chlorbenzol. Gegebenenfalls können die verwendeten Kettenabbrecher und Verzweiger im gleichen Solvens gelöst werden.

Als organische Phase für die Phasengrenzflächenpolykondensation dient beispielsweise Methylenchlorid, Chlorbenzol sowie Mischungen aus Methylenchlorid und Chlorbenzol.

Als wäßrige alkalische Phase dient beispielsweise wäßrige NaOH-Lösung.

Die Herstellung der erfindungsgemäßen Polycarbonate nach dem Phasengrenzflächenverfahren kann in üblicher Weise durch Katalysatoren wie tertiäre Amine, insbesondere tertiäre aliphatische Amine wie Tributylamin, N-Ethylpiperidin oder Triethylamin katalysiert werden; die Katalysatoren können in Mengen von 0,05 bis 10 Mol-%, bezogen auf Mole an eingesetzten Diphenolen, eingesetzt werden. Die Katalysatoren können vor Beginn der Phosgenierung oder während oder auch nach der Phosgenierung zugesetzt werden.

Die Isolierung der erfindungsgemäßen Polycarbonate erfolgt in bekannter Weise.

Die erfindungsgemäßen hochmolekularen, aromatischen Polycarbonate können auch nach dem bekannten Verfahren in homogener Phase, dem sogenannten "Pyridinverfahren" sowie nach dem bekannten Schmelzumesterungsverfahren unter Verwendung von beispielsweise Diphenylcarbonat anstelle von Phosgen hergestellt werden, dies ist aber nicht bevorzugt bzw. nicht empfehlenswert.

Die nach dem erfindungsgemäßen Verwendungsverfahren erhältlichen (Co)Polycarbonate haben bevorzugt Molekulargewichte Mw (Gewichtsmittel, ermittelt durch Gelchromatographie nach vorheriger Eichung) von mindestens 12 000, besonders bevorzugt von 12 000 bis 220 000 und insbesondere von 20 000 bis 100 000. Sie können linear oder verzweigt sein, sie sind Homopolycarbonate oder Copolycarbonate auf Basis der Diphenole der Formel (I).

Gegenstand der Erfindung sind somit auch hochmolekulare, aromatische, thermoplastische (Co)-Polycarbonate mit Mw (Gewichtsmittelmolekulargewichten) von mindestens 12 000, vorzugsweise 12 000 bis 220 000 und insbesondere von 20 000 bis 100 000, die bifunktionelle Carbonatstruktureinheiten der Formel (Ia)

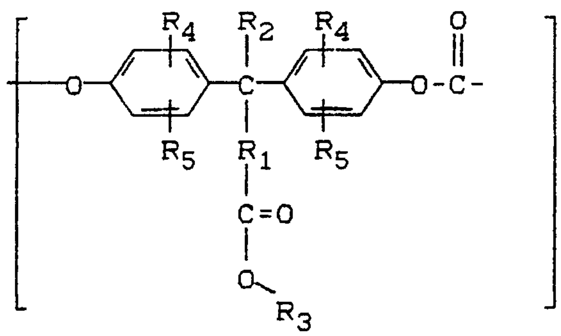

(Ia)

worin

$R_1$, $R_2$, $R_3$, $R_4$, $R_5$ und n die für die Formel (I) genannten Bedeutungen haben,

in Mengen von 100 Mol-% bis 0,5 Mol-%, vorzugsweise in Mengen von 50 Mol-% bis 1 Mol-% und insbesondere in Mengen von 30 Mol-% bis 1,5 Mol-% und ganz besonders 20 Mol-% bis 2 Mol-%, bezogen jeweils auf die Gesamtmenge von 100 Mol-% an difunktionellen Carbonatstruktureinheiten im Polycarbonat, enthalten.

Gegenstand der Erfindung ist fernerhin auch die Verwendung der seitenständig tert.-Alkylestergruppen aufweisenden Bisphenole der Formel (I) sowie gegebenenfalls anderen Diphenolen, z.B. der Formel (VII) und Dicarbonsäuren, eingesetzt in Form ihrer Säurechloride, zur Herstellung von aromatischen Polyestern und insbesondere zur Herstellung von (Co)Polyestercarbonaten auf der Basis der erfindungsgemäßen Bisphenole der Formel (I), gegebenenfalls anderen Diphenolen (c1) der Formel (VII), Dicarbonsäuren oder Gemischen von Dicarbonsäuren, eingesetzt in Form ihrer Säurechloride (Komponente b1)) und gegebenenfalls Phosgen, Diphenylcarbonat oder Bischlorkohlensäureestern. Die hierfür einzusetzenden erfindungsgemäßen Bisphenole der Formel (I) sowie andere, Diphenole (VII) (Komponente c1)) wurden bereits aufgeführt.

Als aromatische Dicarbonsäuren seien insbesondere die Terephthalsäure und Isophthalsäure genannt, ferner auch tert.-Butylisophthalsäure, 3,3'-Diphenyldicarbonsäure, 4,4'-Diphenyldicarbonsäure, 4,4'-Benzophenondicarbonsäure, 3,4'-Benzophenondicarbonsäure, 4,4'-Diphenyletherdicarbonsäure, 4,4'-Diphenylsulfondicarbonsäure, 2,2-Bis-(4-carboxyphenyl)-propan, sowie Trimethyl-3-phenylindan-4,5-dicarbonsäure. In geringen Mengen können als Abbrecher oder Verzweiger auch monofunktionelle bzw. trifunktionelle Ester mitverwendet werden.

Bevorzugt finden die erfindungsgemäßen Bisphenole Verwendung in Verfahren zur Herstellung von (Co)Polyestern und (Co)Polyestercarbonaten, in denen die aromatischen Diphenole zu 0,5 bis 100 Mol-%, bevorzugt 1 is 50 Mol-% und insbesondere 1,5 bis 30, ganz besonders bevorzugt 2 bis 20 Mol-% aus

erfindungsgemäßen Bisphenolen der Formel (I) bestehen. Bevorzugt werden in den erfindungsgemäßen (Co)Polyestern bzw. (Co)Polyestercarbonaten solche, in denen die Dicarbonsäuren in einer Menge von 5 bis 98 Mol-%, bevorzugt 15 bis 95 Mol-%, besonders bevorzugt von 20 bis 50 Mol-% und 60 bis 95 Mol-% und insbesondere von 25 bis 45 Mol-% und 75 bis 90 Mol-%, bezogen auf die Summe aus Dicarbonsäure und der Kohlensäure, enthalten sind.

Die erfindungsgemäßen (Co)Polyester und (Co)Polyestercarbonate können nach Verfahren hergestellt werden, wie sie prinzipiell für die Polyester- bzw. Polyestercarbonat-Herstellung aus der Literatur bekannt sind, so z.B. nach Verfahren in homogener Lösung, nach Schmelzumesterungsverfahren, besonders aber nach dem Zweiphasengrenzflächenverfahren. Bevorzugt werden das Schmelzumesterungsverfahren, insbesondere aber das Zweiphasengrenzflächenverfahren angewandt, da bei letzterem eine besonders schonende Reaktionsweise, bei der die tert.-Alkylesterseitengruppen erhalten bleiben, garantiert ist.

Verfahren zur Herstellung von Polyestern und Polyestercarbonaten in homogener Lösung werden beispielsweise beschrieben in den DE-PS 1 420 475, US-PS 3 169 121 und US-PS 4 156 069 sowie in der Monographie Polymer Reviews, Volume 10, Condensation Polymers by Interfacial and Solution Methods, Paul W. Morgan, Interscience Publishers, New York 1965, Kap. VIII, S. 325 ff. So können beispielsweise Polyester durch Umsetzung von Dicarbonsäuredichloriden mit aromatischen Dihydroxyverbindungen in Gegenwart von Pyridin als Säureacceptor und Pyridin oder chlorierten Kohlenwasserstoffen wie Methylenchlorid und Chlorbenzol als Lösungsmittel hergestellt werden.

(Co)Polyestercarbonate können beispielsweise in homogener Lösung hergestellt werden, indem Phosgen, Dicarbonsäure(chlorid) und aromatische Dihydroxyverbindung oder Phosgen, Dicarbonsäure und aromatische Dihydroxyverbindungen als Monomere eingesetzt werden. Die Reaktion verläuft schon bei niedrigen Temperaturen, so z.B. bei 10 bis 40 °C, ausreichend schnell.

Schmelzumesterungsverfahren zur Herstellung von Polyestern und Polycarbonaten sind beispielsweise das Acetat-Verfahren und insbesondere bevorzugt das Phenylester-Verfahren.

Das Phenylesterverfahren zur Herstellung von aromatischen (Co)Polyestern und (Co)Polycarbonaten wird beispielsweise in den Patentschriften US 4 661 580, 4 680 371, 4 680 372, EP 79 075, EP 146 887, EP 156 103, EP 234 913, EP 234 914, EP 240 301 und DE-AS 1 495 626 und DE-AS 2 232 877 beschrieben. Bei diesem Verfahren werden im allgemeinen bei Temperaturen von >200 °C aromatische Dihydroxyverbindungen, wie z.B. Bisphenol-A mit aromatischen Dicarbonsäureestern, wie Isophthalsäurediphenylester und Terephthalsäurediphenylester, in der Schmelze unter Phenolabspaltung zu aromatischen Polyestern umgesetzt. Zur Herstellung von Polyestern nach diesem Verfahren können auch aromatische Dihydroxyverbindungen, Dicarbonsäuren und Diester der Kohlensäure wie Diphenylcarbonat als Ausgangsstoffe umgesetzt werden. In diesem Fall werden die für die Reaktion benötigten Phenylester der Dicarbonsäuren intermediär unter $CO_2$- und Phenolabspaltung gebildet.

Zur Herstellung von (Co)Polyestercarbonaten nach dem Phenylesterverfahren werden im allgemeinen zusätzlich zu den Phenylestern der Dicarbonsäuren Diester der Kohlensäure wie Diphenylcarbonat, eingesetzt. Es können jedoch auch nur aromatische Dihydroxyverbindungen, Dicarbonsäuren und Diphenylcarbonat für die Polyestercarbonatherstellung eingesetzt werden, Bei der Polyestercarbonatherstellung ist im allgemeinen die Summe aus eingesetzten Diestern von Dicarbonsäuren und Dicarbonsäuren kleiner als die Summe an eingesetzten Bisphenolen, alles bezogen auf Molteile. Es können auch Katalysatoren üblicher Art zur Beschleunigung der Kondensationsreaktion verwendet werden, Hilfslösemittel können eingesetzt werden, eine Festphasenkondensation des Prepolymeren mag durchgeführt werden und ein Abdestillieren von Phenol etc. im Vakuum vorgenommen werden.

Das wegen seiner schonenden Verfahrensweise sehr stark bevorzugte Zweiphasengrenzflächenverfahren zur Herstellung von (Co)Polyestern und (Co)Polyestercarbonaten wird beispielsweise beschrieben in den Patentschriften EP 68 014, 88 322, 134 898, 151 750, 182 189, 219 708 und 272 426 sowie den DE-OSen 2 940 024, 3 007 934 und 3 440 020 sowie in der Monographie, Polymer Reviews, Volume 10, Condensation Polymers by Interfacial and Solution Methods, Paul W Morgan, Interscience Publishers, New York 1965, Kap. VIII, S. 325 ff.

Als Ausgangskomponenten für die Herstellung der erfindungsgemäßen (Co)Polyester und (Co)-Polyestercarbonate nach dem Zweiphasengrenzflächenverfahren dienen bevorzugt aromatische Dihydroxyverbindungen, Dicarbonsäuredichloride und Phosgen.

Auch der Einsatz von Terephthalsäure und/oder Isophthalsäure ist beim Zweiphasengrenzflächenverfahren möglich, wobei intermediär mit Hilfe von Phosgen die Dicarbonsäurechloride bzw. -dichloride gebildet werden.

Die Polykondensation erfolgt beim Zweiphasengrenzflächenverfahren in einem Zweiphasensystem aus wäßrig-alkalischer Lösung und mit Wasser nicht mischbarem organischem Lösungsmittel. Beispielsweise wird die aromatische Dihydroxyverbindung, in wäßrigem Alkali als Diphenolat gelöst, mit dem Dicarbonsäuredichlorid und gegebenenfalls Phosgen, gelöst im organischen Lösungsmittel, unter Rühren umgesetzt. Der sich bildende Polyester bzw. das sich bildende Polyestercarbonat werden im organischen Lösungsmittel gelöst. Wenn Dicarbonsäuredichlorid und Phosgen eingesetzt werden, wenn also Polyestercarbonate hergestellt werden, dann können das Dicarbonsäuredichlorid und das Phosgen auf unterschiedliche Weise eingesetzt werden. So kann beispielsweise zunächst nur Dicarbonsäurechlorid mit dem Alkalibisphenolat umgesetzt werden und dann erst das für die Vollendung der Polykondensation benötigte Phosgen zugesetzt werden. Es können beispielsweise auch das Dicarbonsäuredichlorid und ein Teil des benötigten Phosgens gemeinsam mit dem Bisphenolat umgesetzt werden und dann der Rest der benötigten Phosgengesamtmenge zugesetzt werden. Die Polykondensation wird im allgemeinen mit Hilfe eines Katalysators durchgeführt. Der Katalysator, unten näher beschrieben, kann beispielsweise zugesetzt werden, wenn die Dicarbonsäurechloride und das Phosgen bereits eine gewisse Zeit mit dem Alkalibisphenolat reagiert haben. Er kann jedoch auch schon, zumindest teilweise, gleich zu Beginn der Reaktion eingesetzt werden. Es können aber auch unterschiedliche Katalysatoren in einer Kondensationsreaktion verwendet werden, wobei der Zugabezeitpunkt der einzelnen Katalysatoren unterschiedlich sein kann. Im allgemeinen wird die Polykondensation zur Einstellung bestimmter Molekulargewichte in Gegenwart von Kettenbegrenzern durchgeführt. Zur Erzielung bestimmter Eigenschaften, so z.B. von Strukturviskosität, können beispielsweise Verzweiger eingesetzt werden. Auch Antioxidantien können, z.B. zur Erzielung sehr farbheller Polykondensate, in der Reaktion Einsatz finden.

Als mit Wasser nicht mischbare organische Lösungsmittel können beispielsweise chlorierte Kohlenwasserstoffe, wie Chloroform, Dichlorethan, Tri- und Tetrachlorethylen, Tetrachlorethan, Dichlormethan, Chlorbenzol und Dichlorbenzol, aber auch nicht chlorierte Kohlenwasserstoffe, wie Toluol und Xylol, eingesetzt werden. Bevorzugt werden Chlorbenzol oder Dichlormethan oder Mischungen von beiden verwendet.

Als Kettenbegrenzer werden Verbindungen eingesetzt, wie sie von Polycarbonaten, Polyestern und Polyestercarbonaten her bekannt sind, so z.B. sekundäre Amine, Phenole und Säurechloride. Bevorzugt werden Phenole, wie Phenol und Alkylphenol, besonders bevorzugt solche mit $C_1$-$C_{12}$-Alkylgruppen, wie p-tert.-Butylphenol, m- und p-3,5-Dimethyl-heptyl-phenol und m- und p- 1,1,3,3-Tetramethylbutyl-phenol sowie Hydroxydiphenyl und p-Cumylphenol verwendet. Ganz besonders bevorzugt wird das p-[1,1,3,3-Tetramethylbutyl]-phenol (p-Isooctylphenol) eingesetzt.

Als Verzweiger dienen, falls benutzt, die bereits beschriebenen drei- oder mehrfunktionellen Verbindungen.

Als Katalysatoren können tertiäre Amine und/oder Phasentransferkatalysatoren, wie quartäre Ammonium- und Phosphoniumverbindungen und/oder Kronenether eingesetzt werden. Bevorzugte Katalysatoren sind beispielsweise N-Ethylpiperidin, Tetrabutylammoniumbromid und/oder Triphenylbenzylphosphoniumbromid.

Die erfindungsgemäßen (Co)Polyester und (Co)Polyestercarbonate weisen im allgemeinen mittlere Molekulargewichte $\overline{M}w$ von mindestens 10 000, vorzugsweise von 10 000 bis 250 000 und insbesondere von 15 000 bis 80 000 auf.

Die Isolierung der nach dem bevorzugten erfindungsgemaßen Verfahren erhältlichen (Co)-Polyestercarbonate geschieht in bekannter Weise, indem man die bei Phasengrenzflächenverfahren erhaltene organische Phase abtrennt, neutral und elektrolytfrei wäscht und dann beispielsweise aus organischer Phase unter Zusatz eines Nichtlösemittels fällt und trocknet.

Den erfindungsgemäßen (Co)Polyestercarbonaten können noch vor oder nach ihrer Verarbeitung die für thermoplastische (Co)Polyestercarbonate üblichen Additive wie Stabilisatoren, Entformungsmittel, Pigmente, Flammschutzmittel, Antistatika, Füllstoffe und Verstärkungsstoffe in disperse Fasern, in den üblichen Mengen zugesetzt werden.

Die erfindungsgemäßen aromatischen (Co)Polyester, (Co)Polycarbonate und (Co)Polyestercarbonate können zu Formkörpern verarbeitet werden, indem man beispielsweise die in bekannter Weise isolierten (Co)Polyestercarbonate zu Granulat extrudiert und dieses Granulat gegebenenfalls nach Zusatz von Additiven durch Spritzguß zu verschiedenen Artikeln in bekannter Weise verarbeitet.

Insbesondere werden die erfindungsgemäßen (Co)Polyestercarbonate zur Herstellung von Polymerblends mit verbesserten Eigenschaften (vgl. Le A 27 122) verwendet.

In den nachfolgenden Beispielen B.1 bis B.5 wird die relative Viskosität an 0,5 gew.-%igen Lösungen der Polymeren in $CH_2Cl_2$ bei 25° C gemessen, soweit nicht anders angegeben.

A) Herstellung der neuen Bisphenolverbindungen vom Typ (I)

<u>Beispiel A.1</u>

Herstellung von 4,4-Bis-(4-hydroxyphenyl)-valeriansäure-tert.-butylester (Formel (I), $R_1$ = $-(CH_2)_2$; $R_2$ = $CH_3$; $R_3$ = $-C(CH_3)_3$; $R_4$, $R_5$ = H)

143,2 g (0,5 Mol) 4,4-Bis-(4'-hydroxyphenyl)-valeriansäure werden in 600 ml Methylisopropylketon gelöst, mit 5 ml konz. Schwefelsäure versetzt und auf -7°C abgekühlt. Bei dieser Temperatur wird bis zur Sättigung Isobutylen eingeleitet. Man läßt über Nacht bei Raumtemperatur stehen und sättigt bei -3°C nochmals mit Isobuten. Man läßt zwei Tage bei Raumtemperatur nachreagieren. Die Lösung wird mit 100 ml Ether versetzt, mit 5 %iger Natriumhydrogencarbonatlösung säurefrei gewaschen, dann mit Wasser neutral gewaschen. Man trocknet über Natriumsulfat und fällt das Produkt mit Petrolether aus. Ausbeute: 60,2 g. Schmelzpunkt: 124 bis 129°C. Aus der Mutterlauge kann durch Aufarbeiten ca. 32 g weiteres Produkt isoliert werden.

Beispiel A.1.2

Herstellung von 4,4-Bis-(4-hydroxyphenyl)-valeriansäure-tert.-amylester (Formel (I), $R_1$ = $-(CH_2)_2$; $R_2$ = $CH_3$; $R_3$ = tert.-Amyl; $R_4$, $R_5$ = H)

Wie in Beispiel A.1 wurden 0,5 Mol 4,4-Bis-(4-hydroxyphenyl)-valeriansäure (Herstellung wie in US 2 984 685 beschrieben, Fp 173 - 174°C - wasserfreie Form) in Methylisopropylketonlösung in Gegenwart von 5 ml konz. $H_2SO_4$, durch Einleiten von Isopenten bis zur Sättigung (ca, 0,7 Mol bei -5°C) und 60 Stunden Stehenlassen bei 0°C bis Raumtemperatur in den tert.-Amylester überführt und der Ester wie in A.1 isoliert.

Beispiel A.1.3

Herstellung von 4,4-Bis-(3-methyl-4-hydroxyphenyl)-valeriansäure-tert.-butylester (Formel (I), $R_1$ = $-(CH_2)_2$; $R_2$ = $CH_3$; $R_3$ = tert.-Butyl; $R_4$ = $CH_3$; $R_5$ = H)

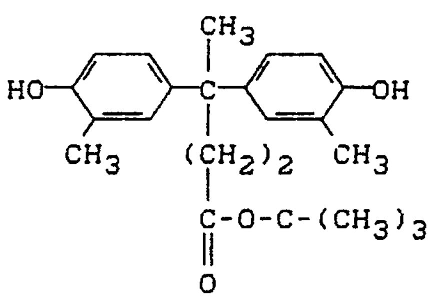 ;

0,5 Mol 4,4-Bis-(3-methyl-4-hydroxyphenyl)-valeriansäure (Fp 144 - 146°C, aus Methanol/Wasser umkristallisiert) werden in 600 ml Methylisopropylketon gelöst, mit 5 ml konz. $H_2SO_4$ versetzt, auf -10°C abgekühlt und mit Isobutylen gesättigt. Nach langsamen Erwärmen auf Raumtemperatur wird nochmals Isobutylen in eine im Eisbad gekühlte Lösung bis zur Sättigung eingeleitet und weitere 2 Tage bei Raumtemperatur stehengelassen. Nach Aufnehmen in Ether und Auswaschen geringer Mengen an Ausgangssäure wird die Etherlösung getrocknet. Nach Einengen wird mit Petrolether ausgefällt und der tert.-Alkylester isoliert.

Beispiel A.1.4

Herstellung von 4,4-Bis-(3,5-dimethyl-4-hydroxyphenyl)-valeriansäure-tert.-butylester

Nach der Verfahrensweise von Beispiel A.1 werden 4,4-Bis-(3,5-dimethyl-4-phenyl)-valeriansäure, Fp. 130 bis 131°C, mit Isobuten unter Schwefelsäurekatalyse in den tert.-Butylester überführt. Langsam kristallisierendes Öl.

Beispiel A.2 (Vergleich)

Herstellung von 4,4-Bis-(4-hydroxyphenyl)-valeriansäuremethylester

171,6 g (0,6 Mol) 4,4-Bis-(4-hydroxyphenyl)-valeriansäure werden in 288 g absolutem Methanol gelöst, mit 6 g konz. Schwefelsäure versetzt und 5 Stunden unter Ausschluß von Feuchtigkeit am Rückfluß gekocht. Anschließend entfernt man den größten Teil des Methanols und gießt den Rückstand auf Eiswasser. Der ausgefallene Feststoff wird einmal mit 200 ml Ether extrahiert und mit 5 %iger Natriumhydrogencarbonatlösung säurefrei gewaschen, dann mit Wasser neutral gewaschen. Man nimmt den Rückstand in Toluol auf, trocknet über Natriumsulfat und entfernt das Lösungsmittel weitgehend. Die Lösung wird auf 0 °C abgekühlt, wobei sich das Produkt abscheidet. Die Mutterlauge wird abdekantiert, nach Trocknung des Produktes im Vakuum erhält man 106,2 g des Methylester.

B) Verwendung von (I) zur Herstellung von Polymeren

Beispiel B.1

Herstellung eines seitenständigen tert.-Alkylestergruppen tragenden (Co)Polycarbonats (erfindungsgemäß)

6,8 g (0,02 Mol) des Diphenols (I) (2,5 Mol-%) gemäß Beispiel A.1, 178,1 g (0,78 Mol) 2,2-Bis-(4-hydroxyphenyl)-propan, 160,0 g (4 Mol) NaOH und 1850 g Wasser werden in einer Inertgas-Atmosphäre unter Rühren gelöst. Dann fügt man eine Lösung von 2,635 g 4-(1,1,3,3-Tetramethylbutyl)-phenol (Kettenabbrecher) in 1385 ml Methylenchlorid zu. In die gut gerührte Lösung wurden bei pH 13 bis 14 und 21 bis 25 °C 138,5 g (1,4 Mol) Phosgen eingeleitet. Danach werden 3,3 ml Ethylpiperidin zugegeben und noch 45 Min. gerührt. Die bisphenolatfreie wäßrige Phase wird abgetrennt, die organische Phase nach Ansäuern mit Phosphorsäure mit Wasser neutral gewaschen und vom Lösungsmittel befreit. Das (Co)-Polycarbonat zeigte eine relative Lösungsviskosität von 1,31.

Beispiel B.2 (erfindungsgemäß)

1,7 g (0,005 Mol) des Diphenols (I) (5 Mol-%) gemäß Beispiel A.1, 21,7 g (0,095 Mol) 2,2-Bis-(4-hydroxyphenyl)-propan, 20,0 g (0,5 Mol) NaOH und 300 g Wasser werden in einer Inertgas-Atmosphäre unter Rühren gelöst. Dann fügt man eine Lösung von 0,329 g Phenol (als Kettenabbrecher) in 224 ml Methylenchlorid zu. In die gut gerührte Lösung wurden bei pH 13 bis 14 und 21 bis 25 °C 17,3 g (0,175 Mol) Phosgen eingeleitet. Danach werden 0,4 ml N-Ethylpiperidin zugegeben und noch 45 Min. gerührt. Die bisphenolatfreie wäßrige Phase wird abgetrennt, die organische Phase nach Ansäuern mit Phosphorsäure mit Wasser neutral gewaschen und vom Lösungsmittel befreit.

Das (Co)Polycarbonat zeigte eine relative Lösungsviskosität von 1,30.

Beispiel B.3

1,0 g (0,003 Mol) des Bisphenols (I) (3 Mol-%) gemäß Beispiel A.1, 17,1 g (0,055 Mol) 1,1-Bis-(4'-hydroxyphenyl)-3,3,5-trimethylcyclohexan, 9,6 g (0,042 Mol) 2,2-Bis-(4'-hydroxyphenyl)-propan, 24,0 g (0,6 Mol) NaOH und 348 g Wasser werden in einer Inertgas-Atmosphäre unter Rühen gelöst. Dann fügt man eine Lösung von 0,516 g 4-(1,1,3,3-Tetramethyl-butyl)-phenol (als Kettenabbrecher) in 260 ml Methylenchlorid zu. In die gut gerührte Lösung werden bei pH 13 bis 14 und 21 bis 25 °C 19,8 g (0,2 Mol) Phosgen eingeleitet. Danach werden 0,1 ml N-Ethylpiperidin zugegeben und noch 45 Min. gerührt. Die bisphenolatfreie wäßrige Phase wird abgetrennt, die organische Phase nach Ansäuern mit Phosphorsäure mit Wasser neutral gewaschen und vom Lösungsmittel befreit.

Das Polycarbonat zeigte eine relative Lösungsviskosität von 1,26.

Beispiel B.4

462 g (1,35 Mol) des Bisphenols (I) (10,8 Mol-% Bisphenol (I)) gemäß Beispiel A.1, 3,447 kg (12,15 Mol) 2,2-Bis-(3,5-dimethyl-4-hydroxyphenyl)-propan (Tetramethylbisphenol A), 6 kg konzentrierte NaOH und 38 l Wasser werden in einer Inertgas-Atmosphäre unter Rühren gelöst. Dann fügt man eine Lösung von 50,8 g Phenol in 29 l Methylenchlorid sowie 43,2 g Tetrabutylammoniumbromid zu. In die gut gerührte Lösung wurden bei pH 13 bis 14 und 20 bis 23 °C 3,12 kg Phosgen eingeleitet, wobei der pH-Wert durch Zugabe von 2,6 l konz. NaOH auf pH 13 gehalten wird. Danach werden 189 ml N-Ethylpiperidin zugegeben und noch 30 Minuten gerührt. Nach Zusatz von 20 l Methylenchlorid wird die bisphenolatfreie wäßrige Phase

abgetrennt, die organische Phase nach Ansäuern mit Phosphorsäure mit Wasser neutral gewaschen und vom Lösungsmittel befreit. Das Polycarbonat zeigt eine relative Lösungsviskosität von 1,386.

Beispiel B.5

(seitenständige tert.-Alkylestergruppen tragendes Copolyester-Copolycarbonat) - erfindungsgemäß

2166 g (9,5 Mol) 2,2-Bis-(4-hydroxyphenyl)-propan, 171,0 g (0,5 Mol) Bisphenol (I) (5 Mol-%) gemäß Beispiel A.1, 1,845 g konz. NaOH und 60 l Wasser werden in einer Inertgas-Atmosphäre unter Rühren gelöst. Es werden 27 l Methylenchlorid zugegeben. Dann fügt man eine Lösung von 741 g (3,65 Mol) Isophthalsäuredichlorid, 741 g (3,65 Mol) Terephthalsäuredichlorid und 113,3 g 4-(1,1,3,3-Tetramethylphenol) in 3 l Methylenchlorid in 2 Minuten unter Rühren zu. Man rührt 30 Minuten bei 25°C. Anschließend werden 3,6 kg konz. NaOH zugegeben. In die gut gerührte Lösung werden bei pH 13 bis 14 und 21 bis 25°C 1,2 kg Phosgen eingeleitet, wobei der pH-Wert durch Zugabe von 200 ml konz. NaOH auf 13 gehalten wird. Nach Zusatz von 14 ml N-Ethylpiperidin wird 30 Minuten gerührt. Die bisphenolatfreie wäßrige Phase wird abgetrennt, mit 15 l Methylenchlorid verdünnt und die organische Phase nach Ansäuern mit Phosphorsäure mit Wasser neutral gewaschen und vom Lösungsmittel befreit. Das Polyestercarbonat zeigte eine relative Lösungsviskosität von 1,258.

Beispiel B.6

(seitenständige tert.-Alkylestergruppen tragendes Copolyester-Copolycarbonat)

2052 g (9,0 Mol) 2,2-Bis-(4-hydroxyphenyl)-propan, 342 g (1 Mol) Bisphenol (II) (10 Mol-%), 1,845 g konz. NaOH und 60 l Wasser werden in einer Inertgas-Atmosphäre unter Rühren gelöst. Es werden 27 l Methylenchlorid zugegeben. Dann fügt man eine Lösung von 741 g (3,65 Mol) Isophthalsäuredichlorid, 741 g (3,65 Mol) Terephthalsäuredichlorid und 113,3 g 4-(1,1,3,3-Tetramethylphenol) in 3 l Methylenchlorid in 2 Minuten unter Rühren zu. Man rührt 30 Minuten bei 25°C. Anschließend werden 3,6 g konz. NaOH zugegeben. In die gut gerührte Lösung wurden bei pH 13 bis 14 und 21 bis 25°C 1,2 kg Phosgen eingeleitet. Nach Zusatz von 500 ml konzentrierter NaOH und 14 ml N-Ethylpiperidin wurde 30 Min. gerührt. Die bisphenolatfreie wäßrige Phase wird abgetrennt, mit 15 l Methylenchlorid verdünnt und die organische Phase nach Ansäuern mit Phosphorsäure mit Wasser neutral gewaschen und vom Lösungsmittel befreit. Das Polyestercarbonat zeigte eine relative Lösungsviskosität von 1,271.

Beispiel B.7

(Vergleichsbeispiel - Polycarbonat mit primären Estergruppen)

0,6 g (0,02 Mol) des Bisphenols (I) gemäß Beispiel A.2, 22,4 g (0,098 Mol) 2,2-Bis-(4-hydroxyphenyl)-propan, 20,0 g (0,5 Mol) NaOH und 288 g Wasser werden in einer Inertgas-Atmosphäre unter Rühren gelöst. Dann fügt man eine Lösung von 0,329 g Phenol in 172 ml Methylenchlorid zu. In die gut gerührte Lösung wurden bei pH 13 bis 14 und 21 bis 25°C 17,3 g (0,175 Mol) Phosgen eingeleitet. Danach werden 0,4 ml Ethylpiperidin zugegeben und noch 45 Min. gerührt. Die bisphenolatfreie wäßrige Phase wird abgetrennt, es werden 100 ml Methylenchlorid zugegeben, die organische Phase nach Ansäuern mit Phosphorsäure mit Wasser neutral gewaschen und vom Lösungsmittel befreit.

Das Polycarbonat zeigte eine relative Lösungsviskosität ≥1,44.

Das Vergleichsbeispiel zeigt, daß hochmolekulare, verzweigte Produkte entstanden sind.

**Patentansprüche**

1. Bisphenole mit tert.-Alkylesterfunktionen der Formel (I)

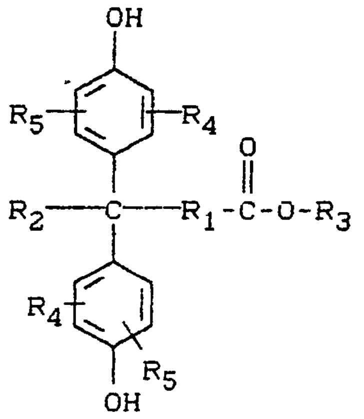

(I),

wobei

$R_1$ ein geradkettiger oder verzweigter $C_1$-$C_6$-Alkylenrest, bevorzugt

$$-(CH_2)_n-;$$

$R_2$ ein $C_1$-$C_{12}$-Alkylrest,
$R_3$ ein tertiärer $C_4$-$C_9$-Alkylrest,
$R_4$, $R_5$ unabhängig voneinander Wasserstoff, Halogen, $C_1$-$C_6$-Alkyl, Cycloalkyl, Aryl und
n eine ganze Zahl von 0 bis 6, insbesondere 2

bedeuten.

**2.** Bisphenole nach Anspruch 1, wobei in Formel (I)
$R_1$ ein $-(CH_2)_n$-Rest mit n = 1-3,
$R_2$ ein Methylrest,
$R_3$ ein tert.-butyl-, tert.-amyl- oder tert.-octyl-Rest und
$R_4$ und $R_5$ unabhängig voneinander H oder $CH_3$ ist.

**3.** 4,4-Bis-(4-hydroxyphenyl)-valeriansäure-tert.-butylester.

**4.** Verfahren zur Herstellung der Bisphenole (I) mit seitenständigen tert.-Alkylestergruppen gemäß Anspruch 1, dadurch gekennzeichnet, daß man Phenole mit tert.-Ketocarbonsäureestern im Molverhältnis (V):(VI) von 2:1 bis 10:1 bei Temperaturen von - 30 °C bis 300 °C und bei Drücken von 1 bis 20 bar in Anwesenheit saurer Katalysatoren und gegebenenfalls in Anwesenheit von Co-Katalysatoren und/oder Lösungsmitteln und/oder wasserentziehenden Mitteln umsetzt.

**5.** Verfahren nach Anspruch 4, dadurch gekennzeichnet, daß man Phenole mit Ketocarbonsäure-tert.-alkylestern im Molverhältnis von 2,5:1 bis 6:1 bei Temperaturen von -15 °C bis 150 °C und bei Drücken von 1 bis 10 bar umsetzt.

**6.** Verfahren zur Herstellung der Bisphenole (I) mit seitenständigen tert.-Alkylestergruppen gemäß Anspruch 1, dadurch gekennzeichnet, daß man Bisphenolcarbonsäuren mit $\alpha,\alpha$-disubstituierten Olefinen bei Temperaturen zwischen -30 °C und 200 °C, vorzugsweise zwischen -15 °C und 120 °C und bei Drücken von 1 bis 100 bar, vorzugsweise von 1 bis 50 bar, in Anwesenheit saurer Katalysatoren und gegebenenfalls in Anwesenheit von Lösungsmitteln und/oder wasserentziehenden Mitteln umsetzt.

**7.** Verwendung der Diphenole der Formel (I) des Anspruchs 1 zur Herstellung von hochmolekularen, aromatischen (Co)Polycarbonaten, (Co)Polyestern oder (Co)Polyestercarbonaten in einem Verfahren zur Herstellung von hochmolekularen, aromatischen Polycarbonaten aus Diphenolen, gegebenenfalls Kettenabbrechern und gegebenenfalls Verzweigern nach den bekannten Methoden der Polycarbonatherstellung, vorzugsweise nach dem Zweiphasengrenzflächenverfahren, dadurch gekennzeichnet, daß man als Bisphenole solche der Formel (I) des Anspruchs 1 in Mengen von 100 Mol-%. bis 2 Mol-%, bezogen jeweils auf die Gesamtmolmenge an eingesetzten Diphenolen, verwendet.

8. Verfahren zur Herstellung von hochmolekularen, aromatischen Polyestercarbonaten, Polyestern aus Diphenolen, gegebenenfalls Kettenabbrechern und gegebenenfalls Verzweigern nach den bekannten Methoden der Polyestercarbonat- bzw. Polyesterherstellung, vorzugsweise nach dem Zweiphasengrenzflächenverfahren, dadurch gekennzeichnet, daß man als Diphenole solche der Formel (I) des Anspruchs 1 in Mengen von 100 Mol-% bis 2 Mol-%, bezogen jeweils auf die Gesamtmolmenge an eingesetzten Diphenolen, verwendet.

9. Hochmolekulare, aromatische Polycarbonate mit Mw (Gewichtsmittelmolekulargewichten) von mindestens 12 000, erhältlich nach dem Verfahren des Anspruchs 5.

10. Hochmolekulare, aromatische Polyestercarbonate, Polyester mit Mw (Gewichtsmittelmolekulargewichten) von mindestens 12 000, erhältlich nach dem Verfahren des Anspruchs 6.